# EUROPEAN PATENT APPLICATION

(11) **EP 1 357 126 A1**
(43) Date of publication of application: **29.10.2003**
(21) Application number: 01272916.6
(22) Date of filing: 28.12.2001
(51) Int. Cl.: C07F 9/10, C07F 9/12, C12N 15/55, C12N 9/20, C12N 15/63, C12N 1/15, C12N 1/19, C12N 1/21, C12N 5/10, C12Q 1/34, G01N 33/50, G01N 33/15

(54) **METHOD OF ASSAYING LYSOPHOSPHOLIPASE D ACTIVITY**

(30) Priority: 28.12.2000 JP 2000403530; 31.01.2001 JP 2001023784
(71) Applicant: Azwell Inc., Osaka-shi, Osaka 540-8575 (JP)
(72) Inventor: KISHIMOTO, Tatsuya, Kobe-shi, Hyogo 658-0025 (JP); SODA, Yasuji, Kobe-shi, Hyogo 658-0003 (JP); MATSUYAMA, Yoshiko, Takatsuki-shi, Osaka 569-0854 (JP); ARAI, Hiroyuki, Bunkyo-ku, Tokyo 112-0002 (JP); AOKI, Junken, Setagaya-ku, Tokyo 154-0003 (JP)
(74) Representative: Helbing, Jörg, Dr. Dipl.-Chem.
(86) International application number: JP0111612
(87) International publication number: WO02053569

(57) **Abstract**

The present invention provides a lysophospholipid of the formula (I) 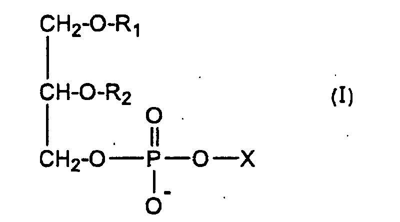 wherein R₁ and R₂ are different from each other and one of them is a saturated or unsaturated fatty acid residue having 8 to 22 carbon atoms, and the other is a hydrogen atom or an acetyl group, and X is a chromophore, a method of assaying a lysophospholipase D activity using the phospholipid as a substrate, a method of screening a substance that inhibits LPLD activity and a reagent and a reagent kit useful for these methods. By the use of the novel lysophospholipid of the present invention as a substrate, a specific and high sensitivity LPLD activity assay becomes attainable. Moreover, by the use of a safe and conveniently detectable chromophore, a number of samples can be assayed rapidly.

## Description

### TECHNICAL FIELD

The present invention relates to a novel lysophospholipid, a reagent containing the lysophospholipid for assaying lysophospholipase D activity, and an assay method of lysophospholipase D activity, which is characterized by the use of the lysophospholipid. The present invention further relates to a screening method of a substance that inhibits lysophospholipase D activity utilizing said method and a kit therefor.

### BACKGROUND ART

Phospholipase D (hereinafter to be also referred to as PLD) is an enzyme that cleaves a bond on the opposite side from the glycerol frame, from among the phosphodiester bonds of phospholipid. From among PLDs, those that particularly utilize lysophospholipid as a substrate are distinguished and referred to as lysophospholipase D (hereinafter to be also referred to as LPLD). Heretofore, the presence of LPLD in the tissues of animals has been reported (Wykle RL et al., Arch. Biochem. Biophys. 184, 149-155; 1977, Kawasaki T. et al., Biochim. Biophys. Acta 920, 85-93, 1987), and_further the presence in not only in the tissues but in plasma of rats (Tokumura A. et al., Lipids 33, 1009-1015, 1998) and in human serum (Akira Tokumura et al., Proceeding of Japanese Conference on the Biochemistry of Lipids 42, 41-44, 2000) has been reported. However, there has been no report on successful purification or cloning of LPLD (Liscovitch M. et al., Biochem. J. 345, 401-415, 2000).

LPLD is considered to mainly utilize lysophosphatidylcholine (hereinafter to be also referred to as LPC) and the like in the body as a substrate and contribute to the production of lysophosphatidic acid (hereinafter to be also referred to as LPA). In recent years, LPA has been shown to have various physiological activities, such as promotion of cell growth, smooth muscle contraction, promotion of tumor cell invasion and the like (Koji Bando et al., Experimental Medicine 18, 184-191, 2000). In addition, LPA has been drawing attention as a diagnostic marker of ovarian cancer, endometrial cancer, cervical cancer (Xu Y. et al., JAMA 280, 719-723, 1998), arteriosclerosis diseases (Siess W. et al., Proc. Natl. Acad. Sci. USA 96, 6931-6936, 1999) and the like, and assay of the activity of LPLD that produces LPA is considered to be extremely important for the diagnoses of these diseases. Moreover, inhibition of LPA receptor has been suggested to be one means of prophylaxis and treatment of atherosclerotic diseases and cardiovascular diseases (Siess W. et al., *ibid.,* 1999). This also indicates a potential use of a substance that inhibits LPLD (LPLD activity inhibitor) capable of producing LPA, for the prophylaxis and treatment of these diseases.

As a method of assaying LPLD activity, there has been known a method comprising mixing a radio-labeled LPC with a sample, and after reaction for a given time, extracting a lipid, separating by thin-layer chromatography and determining the radioactivity of a band thereof. However, this method is not practical.

On the other hand, as an assay method of enzyme activity, which comprises adding a chromophore to a substrate reaction site and measuring a chromogenic compound released by enzymolysis, there have been known a method of assaying an alkaline phosphatase activity using p-nitrophenylphosphoric acid as a substrate, a method of assaying a phospholipase D activity using phosphatidyl-p-nitrophenol as a substrate (Arrigo P. D' et al., Analytica Chimica Acta 304, 249-254, 1995, US Patent No. 5011964) and the like. However, no report has been presented on an assay method of a lysophospholipase D activity.

### DISCLOSURE OF THE INVENTION

It is therefore an object of the present invention to provide a method of specifically and conveniently assaying a lysophospholipase D (LPLD) activity in a sample and a substrate useful for this assay. Another object of the present invention is to screen a substance that inhibits LPLD activity utilizing this assay method and to provide a reagent and a kit useful for the screening.

The present inventors have conducted intensive studies in an attempt to achieve the above-mentioned objects and found a method of assaying an LPLD activity, which comprises using a chromophore-added substrate and assaying the color development using a released chromogenic compound as an index. Moreover, they have succeeded in obtaining a molecular biological finding relating to LPLD heretofore unreported, and based on such finding, confirmed the usefulness of the activity assay method and the screening method, which resulted in the completion of the present invention.

Accordingly, the present invention provides the following.
(1) A lysophospholipid of the formula (I) 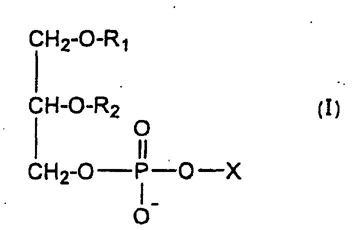 wherein R₁ and R₂ are different from each other and one of them is a saturated or unsaturated fatty acid residue having 8 to 22 carbon atoms, and the other is a hydrogen atom or an acetyl group, and X is a chromophore.
(2) The lysophospholipid of the above-mentioned (1), wherein the fatty acid residue is selected from the group consisting of a linoleic acid residue, an oleic acid residue, a palmitic acid residue and a myristic acid residue.
(3) The lysophospholipid of the above-mentioned (1) or (2), wherein the chromophore is a p-nitrophenyl group or an α-naphthyl group.
(4) A reagent for assaying a lysophospholipase D activity, which comprises the lysophospholipid of the above-mentioned (1) as an active ingredient.
(5) A method of assaying a lysophospholipase D activity of a sample, comprising adding the lysophospholipid of the above-mentioned (1) to the sample to allow reaction of the lysophospholipid and lysophospholipase D in the sample and colorimetrically quantitatively determining an amount of a chromogenic compound released by the reaction.
(6) The assay method of the above-mentioned (5), wherein the sample is selected from the group consisting of whole blood, serum, plasma, urine and saliva.
(7) The assay method of the above-mentioned (6), which is used for the diagnosis of at least one kind of disease selected from the group consisting of ovarian cancer, cervical cancer, endometrial cancer, breast cancer and an arteriosclerotic disease.
(8) A kit for assay of a lysophospholipase D activity of a sample, comprising the lysophospholipid of the above-mentioned (1).
(9) The kit of the above-mentioned (8), wherein the sample is selected from the group consisting of whole blood, serum, plasma, urine and saliva.
(10) The kit of the above-mentioned (8), which is used for the diagnosis of at least one kind of disease selected from the group consisting of ovarian cancer, cervical cancer, endometrial cancer, breast cancer and an arteriosclerotic disease.
(11) A gene comprising a DNA of the following (a) or (b):
   (a) a DNA encoding a protein having a lysophospholipase D activity, which consists of a base sequence depicted in SEQ ID No: 1,
   (b) a DNA encoding a protein having a lysophospholipase D activity, which hybridizes with a DNA consisting of the base sequence of (a) under stringent conditions.
(12) A protein of the following (a) or (b):
   (a) a protein having a lysophospholipase D activity, which consists of an amino acid sequence depicted in SEQ ID No:2,
   (b) a protein having a lysophospholipase D activity, which consists of an amino acid sequence of (a) wherein one to several amino acids have been deleted, substituted or added.
(13) A recombinant vector comprising the gene of the above-mentioned (11).
(14) An expression vector functionally comprising the gene of the above-mentioned (11).
(15) A transformant obtained by transforming a host cell with the vector of the above-mentioned (13) or (14).
(16) A method of producing lysophospholipase D, which comprises culturing, under conditions that permit expression of a protein, a host cell transformed with the expression vector of the above-mentioned (14) and harvesting a substance having a lysophospholipase D activity from the obtained culture.
(17) A lysophospholipase D produced by the production method of the above-mentioned (16).
(18) The reagent for assaying a lysophospholipase D activity of the above-mentioned (4), wherein the lysophospholipase D is a protein encoded by the gene of the above-mentioned (11) .
(19) The reagent for assaying a lysophospholipase D activity of the above-mentioned (4), wherein the lysophospholipase D is the protein of the above-mentioned (12).
(20) A control serum for a lysophospholipase D activity assay, which is obtained by adding a protein encoded by the gene of the above-mentioned (11) to a serum.
(21) A control serum for a lysophospholipase D activity assay, which is obtained by adding the protein of the above-mentioned (12) to a serum.
(22) A method for screening a substance that inhibits a lysophospholipase D activity, which comprises at least the following steps:
   (i) a step for adding a test substance to a sample containing lysophospholipase D,
   (ii) a step for adding the lysophospholipid of the above-mentioned (1) to a sample containing lysophospholipase D,
   (iii) a step for colorimetrically quantitatively determining an amount of a chromogenic compound released by reacting the lysophospholipid and lysophospholipase D in a sample, and
   (iv) a step for comparing the lysophospholipase D activity shown by the degree of color development with that when a test substance is not added.
(23) The screening method of the above-mentioned (22), wherein the lysophospholipase D is encoded by the gene of the above-mentioned (11).
(24) The screening method of the above-mentioned (22), wherein the lysophospholipase D is the protein of the above-mentioned (12).
(25) A kit for screening a substance that inhibits a lysophospholipase D activity, which comprises the lysophospholipid of the above-mentioned (1) and a sample containing lysophospholipase D.
(26) The kit of the above-mentioned (25), wherein the lysophospholipase D is a protein encoded by the gene of the above-mentioned (11).
(27) The kit of the above-mentioned (25), wherein the lysophospholipase D is the protein of the above-mentioned (12).

### BRIEF DESCRIPTION OF THE DRAWINGS

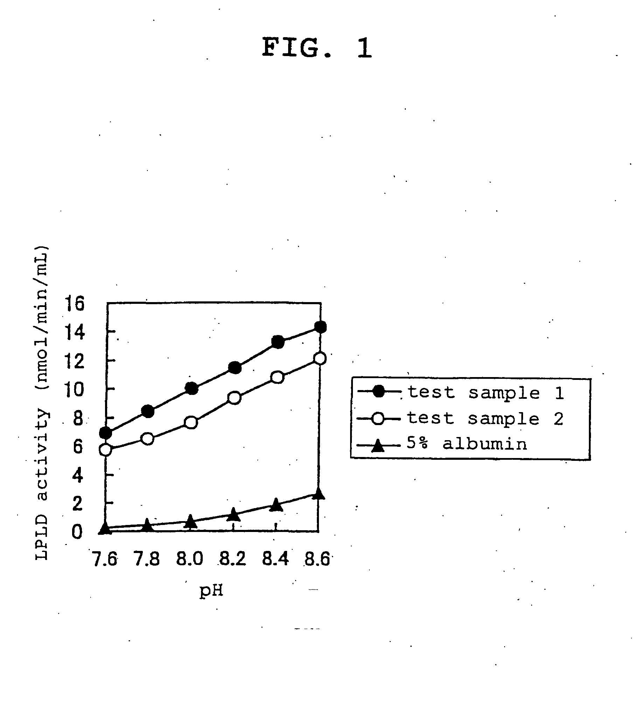

Fig. 1 is a graph showing the results of LPLD activity assay method of the present invention applied to serum samples and 5% serum albumin solutions at various reaction pHs, wherein test sample 1 shows CONSERA and test sample 2 shows Lipid Serum II.

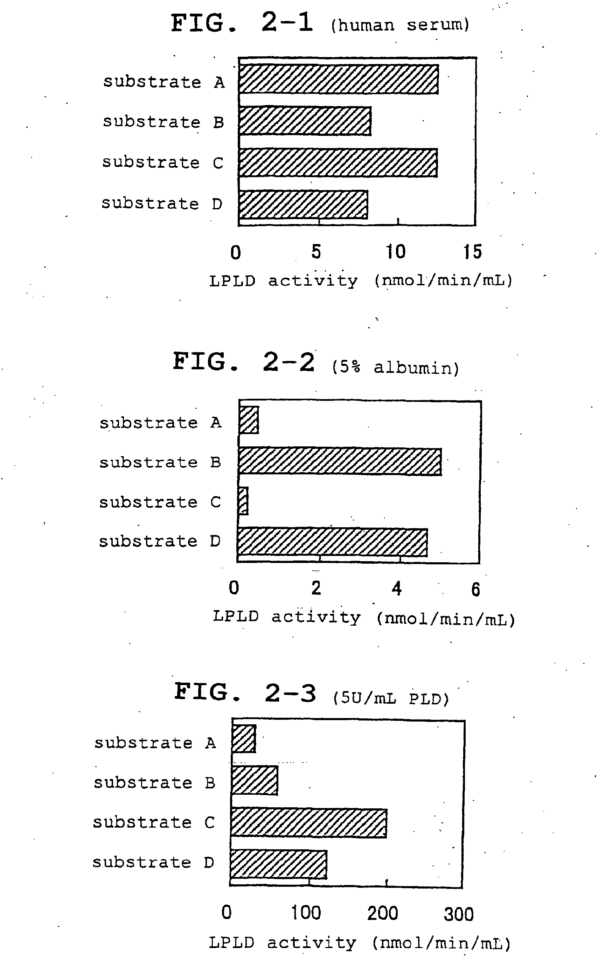

Fig. 2 is a graph showing the results of LPLD activity assay method of the present invention applied to human serum samples, 5% serum albumin solutions and PLD 5U/mL solutions using various substrates, wherein Fig. 2-1 shows the results of human serum sample, Fig. 2-2 shows the results of 5% serum albumin solution and Figure 2-3 shows the results of PLD 5U/mL solution.
   Substrate A: soybean-derived 1-hydroxy-2-linoleoyl-phosphatidyl-p-nitrophenol
   Substrate B: soybean-derived 1-linoleoyl-2-hydroxy-phosphatidyl-p-nitrophenol
   Substrate C: yolk-derived 1-hydroxy-2-oleoyl-phosphatidyl-p-nitrophenol
   substrate D: yolk-derived 1-oleoyl-2-hydroxy-phosphatidyl-p-nitrophenol
Fig. 3 is a graph showing the results of LPLD activity assay method of the present invention applied to human serum samples and 5% serum albumin solutions using various substrate concentrations.
Fig. 4 is a graph showing the results of LPLD activity assay method of the present invention applied to serially diluted human sera as samples.
Fig. 5 is a graph showing the results of LPLD activity assay method of the present invention applied to purified LPLD (LPLD purification product) as a sample at various reaction pHs, wherein the substrate used was 1-myristoyl-phosphatidyl-p-nitrophenol.

### DETAILED DESCRIPTION OF THE INVENTION

As used in the present invention, LPLD is an enzyme that cleaves a bond on the opposite side from the glycerol frame, from among the phosphodiester bonds of phospholipid. Specifically, it is a protein encoded by the gene comprising the DNA of the following (a) or (b), or a protein of the following (c) or (d):
(a) a DNA encoding a protein having a lysophospholipase D activity, which consists of a base sequence depicted in SEQ ID No:1,
(b) a DNA encoding a protein having a lysophospholipase D activity, which hybridizes with a DNA consisting of the base sequence of (a) under stringent conditions,
(c) a protein consisting of an amino acid sequence depicted in SEQ ID No:2,
(d) a protein having a lysophospholipase D activity, which consists of the amino acid sequence of (c) , wherein one to several amino acids have been deleted, substituted or added.

Particularly, LPLD of the present invention is preferably a protein having the following 3 amino acid sequences:

(i) MHTARVRDIEHLTSLDFFRK (SEQ ID NO:3)

(ii) YASERNGVNVISGPIFDYDYDGLHDTEDK (SEO ID NO:4)

(iii) FNHRWWGGQPLWITATK (SEQ ID NO:5)

In the present invention, lysophospholipase D is not subject to any particular limitation as regards its derivation as long as it has a lysophospholipase D activity, and encompasses any such as one having a biological origin and naturally present, a natural or artificial mutant, one derived from a transformant obtained by introducing an exogenous lysophospholipase D gene, and the like.

The method for artificially preparing a mutant includes, for example, a site-directed mutagenesis. More specifically, a mutant lysophospholipase D can be obtained by causing an optional mutation in the base sequence depicted in SEQ ID No:1 by this method. A mutant lysophospholipase D thus obtained has a lysophospholipase D activity to cleave the bond on the opposite side from the glycerol frame, from among the phosphodiester bonds of lysophospholipid.

The lysophospholipase D in the present invention can be obtained by an appropriate known method such as (1) a method of isolation and purification of a culture of a cell or tissue that produces the enzyme as a starting material, or a biological sample such as whole blood, serum, plasma, urine, saliva and the like, (2) a chemical synthesis method or (3) purification from a cell engineered to express lysophospholipase D by a genetic recombination technique etc. and the like.

The isolation and purification of lysophospholipase D in the present invention can be done as follows. That is, a cell that expresses lysophospholipase D is cultured in a suitable liquid medium and the obtained culture is extracted and purified by a known method. For the extraction and purification, a suitable known method is used depending on the fraction containing the objective product.

A specific procedure includes the following. First, a culture is subjected as it is to a conventional method, such as filtration, centrifugal separation and the like, and the cell or a supernatant is recovered. When the enzyme is accumulated in the cell, the recovered cell is suspended in a suitable buffer, and the surfactant is added at a suitable concentration to solubilize the membrane. The obtained crude extract solution is processed by generally-employed methods in a suitable combination, in the presence of a surfactant where necessary, whereby the enzyme is isolated and purified.

When the enzyme is present in a culture medium, the culture is first subjected to filtration or centrifugal separation and the sediment (solid such as cell etc.) is removed to give a solution portion alone, which is processed by generally-employed methods in a suitable combination to isolate and purify the objective substance. There are mentioned methods utilizing solubility such as salting out, solvent precipitation method and the like, methods utilizing difference in molecular weights such as dialysis, ultrafiltration, gel filtration, SDS-PAGE and the like, methods utilizing charge such as ion exchange chromatography and the like, methods utilizing specific affinity such as affinity chromatography and the like, methods utilizing difference in hydrophobicity such as reverse high performance liquid chromatography and the like, methods utilizing difference in isoelectric point such as isoelectric focusing and the like, and the like. More specifically, for example, the enzyme can be separated and purified by a conventional method such as concentration under reduced pressure, lyophillization, extraction using a conventional solvent, pH adjustment, treatment with conventional adsorbent such as anion exchange resin, cation exchange resin, non-ionic adsorbent resin and the like, crystallization, recrystallization and the like.

The production of lysophospholipase D in the present invention by chemical synthesis can be performed by, for example, synthesis or semi-synthesis of the amino acid by a peptide synthesizer based on the amino acid sequence depicted in SEQ ID NO:2.

In the present invention, moreover, LPLD can be also obtained by subjecting a naturally occurring biological sample such as whole blood, serum, plasma and the like to a purification step. For example, LPLD can be obtained by subjecting a sample containing LPLD to the following steps.
step 1: polyethylene glycol precipitation
step 2: affinity chromatography using blue sepharose 6FF column
step 3: affinity chromatography using concanavalin A sepharose
step 4: affinity chromatography using BioAssistQ column
step 5: affinity chromatography using HiTrap Heparin column
step 6: hydrophobic chromatography using RESOURCE PHE column
step 7: hydroxyapatite chromatography using Econo-Pac CHT-II cartridge.

The level of purification in each step can be confirmed through the presence of a protein as shown by the number and intensity of the peaks detectable by HPLC, the number and density of bands detectable by SDS-PAGE, or by using LPLD activity as an index.

The LPLD activity can be assayed by, for example, utilizing a reaction of LPLD to produce lysophosphatidic acid and choline using lysophosphatidylcholine as a substrate, and treating lysophosphatidylcholine with each fraction obtained in each step, and assaying the amount of choline produced thereby. Alternatively, the LPLD activity assay method of the present invention to be mentioned below may be used.

Cloning of the lysophospholipase D gene is generally performed by the following method. First, the enzyme is completely or partially purified by a general method from a sample containing lysophospholipase D, and an N-terminal amino acid sequence is determined by the Edman method. An oligonucleotide having a base sequence corresponding to the determined partial amino acid sequence is synthesized, and using this as a primer or probe, the gene is cloned from a cDNA or genomic DNA library already present, which has been prepared from a cell or tissue that produces lysophospholipase D.

Alternatively, an antibody to the enzyme or its subunit is prepared according to a conventional method using the entirety or a part of the completely or partially purified lysophospholipase D as an antigen, and a DNA encoding the enzyme can be cloned by antibody screening from cDNA or genomic DNA library prepared from a cell or tissue that produces lysophospholipase D.

Different from the aforementioned method, a DNA encoding lysophospholipase D in the present invention can be also cloned directly by PCR. That is, PCR is performed according to a conventional method using, as a template, a genomic DNA or cDNA (or mRNA) derived from a cell or tissue having the enzyme activity, and using a pair of suitable oligonucleotides, whose amplification fragment covers a coding region of lysophospholipase D, as a primer, whereby a DNA fragment containing the coding region of the enzyme can be amplified.

The base sequence of the obtained DNA insert can be determined by a known sequencing technique such as Maxam-Gilbert method, dideoxy termination method and the like.

The gene encoding lysophospholipase D in the present invention includes the entirety or a part of the DNA substantially consisting of the base sequence depicted in SEQ ID NO:1. As used herein, by the "DNA substantially consisting of" is meant, in addition to the DNA consisting of the above-mentioned particular base sequence, a DNA consisting of a base sequence capable of hybridizing with the DNA consisting of the above-mentioned particular base sequence under the stringent conditions (in the present invention, the conditions under which a DNA having a homology of not less than about 60%, preferably about 80%, more preferably not less than about 90%, with the base sequence can hybridize, wherein the stringency can be controlled by suitably changing the temperature, salt concentration and the like during hybridization reaction and washing).

The gene of the present invention can be obtained by any method. For example, complementary DNA (cDNA) prepared from mRNA, genomic DNA prepared from genomic library, chemically synthesized DNA, a DNA obtained by amplification by PCR using RNA or DNA as a template, a DNA constructed by suitably combining these methods and the like can be mentioned.

The present invention also relates to a recombinant vector containing the above-mentioned gene. The recombinant vector in the present invention is not particularly limited as long as it is capable of replication-retention or autonomous growth in various hosts of procaryotic cells and/or eucaryotic cells, and includes plasmid vector, phage vector and the like. The recombinant vector can be prepared by inserting any of the above-mentioned DNAs into a cloning vector or expression vector available in the pertinent field, conveniently using a suitable restriction enzyme site.

Particularly, the recombinant vector of the present invention is an expression vector functionally containing a gene encoding lysophospholipase D. As used herein, by the "functionally" is meant that the gene (DNA) is transcribed in the host cell compatible with the vector and the gene is located such that the protein encoded thereby can be produced. Preferably, it is a vector having an expression cassette sequentially arranging a promoter region, an initiating codon, a gene encoding lysophospholipase D, a termination codon and a terminator region. The recombinant vector of the present invention is not particularly limited as long as it is capable of replication-retention or autonomous growth in various hosts of procaryotic cells and/or eucaryotic cells, and includes plasmid vector, phage vector and the like.

The recombinant vector can be conveniently prepared by functionally ligating a DNA encoding lysophospholipase D in the present invention with an expression vector available in the pertinent field by a conventional method. The expression vector to be used is not particularly limited as long as it expresses and produces lysophospholipase D in various hosts of procaryotic cells and/or eucaryotic cells, but preferably contains a selective marker gene for selection of transformant. When, for example, a mammalian cell is to be transformed, a plasmid obtained by inserting a selective marker gene (neomycin resistance gene, dihydrofolate reductase etc.}, which derived from a plasmid such as pSV2-neo, pSV2-dhfr and the like, into a plasmid to which a promoter of an animal virus such as SV40, RSV, MMLV and the like and a polyadenylation signal are ligated via a restriction enzyme site, preferably multicloning site, can be used.

The transformed cell in the present invention can be prepared by introducing an expression vector containing the aforementioned DNA encoding lysophospholipase D into a host cell. The host cell is not particularly limited as long as it is compatible with the expression vector to be used and can be transformed, and various cells generally used in the technical field of the present invention, such as natural cell, artificially established recombinant cell and the like, can be used. To be specific, bacteria such as Escherichia coli, Bacillus subtilis and the like, fungus such as yeast and the like, animal cell, insect cell and the like can be used. Preferably, mammalian cell, particularly rat-derived cell, hamster-derived cell (CHO, BHK etc.), mouse-derived cell (COP, L, C127, Sp2/0, NS-1, NIH T3 etc.), monkey-derived cell (COS1, COS3, COS7, CV1, Velo etc.) and human-derived cell (Hela, diploid fibroblast derived cell, myeloma, Namalwa etc.) are used.

The introduction of an expression vector into a host cell can be done by a conventionally known method. For example, for introduction into a mammalian cell, calcium phosphate coprecipitation method, protoplast fusion method, microinjection method, electroporation method, lysosome method and the like can be used.

The lysophospholipase D in the present invention can be also produced by culturing the transformant containing an expression vector prepared as mentioned above. The medium preferably contains a carbon source and an inorganic or organic nitrogen source necessary for the growth of the host cell (transformant). The carbon source is, for example, glucose, dextrin, soluble starch, sucrose and the like, and the nitrogen source is, for example, ammonium salt, nitrate, amino acid, corn steep liquor, peptone, casein, meat extract, soybean cake, potato extract and the like. When desired, other nutrient source [e.g., inorganic salt (calcium chloride, sodium dihydrogen phosphate, magnesium chloride etc.), vitamin, antibiotics (tetracycline, neomycin, kanamycin, ampicillin etc.)] may be contained.

The culture is conducted according to a method known in the pertinent field. The culture conditions should permit expression of a protein and, for example, temperature, pH of medium and culture time are appropriately determined to permit production of an enzyme in large amounts.

For example, when the host is an animal cell, minimum essential medium (MEM), Dulbecco's Modified Eagle's Medium (DMEM), RPMI-1640 medium, 199 medium and the like, containing about 5-20% of fetal calf serum (FCS), can be used as the medium. The pH of the medium is preferably about 6-8, and the culture is generally conducted at 30-40°C for about 15-72 hours, with aeration and stirring where necessary.

The lysophospholipase D in the present invention can be harvested from the culture obtained by the above-mentioned culture in the same manner as in the aforementioned isolation and purification from a cell expressing lysophospholipase D from the culture.

The thus-obtained purified LPLD or recombinant LPLD has a substrate specificity as shown in, for example, Table 1.

**Table 1**

| substrate | Reaction product |
|---|---|
| lysophosphatidylcholine | lysophosphatidic acid, choline |
| lysophosphatidylserine | lysophosphatidic acid, serine |
| lysophosphatidylethanolamine | lysophosphatidic acid, ethanolamine |
| lysophosphatidylinositol | lysophosphatidic acid, inositol |
| lysosphingomyelin | Sphingosine-1-phosphate, choline |
| 1-alkyl-2-hydroxy-glycerophosphocholine (lysoPAF) | 1-alkyl-2-hydroxy-glycero-3-phosphate, choline |
| 1-alkyl-2-acetyl-glycerophosphocholine (PAF) | 1-alkyl-2-acetyl-glycero-3-phosphate, choline |
| 1-alkenyl-2-hydroxy-glycerophosphocholine (lysoplasmalogen) | 1-alkenyl-2-hydroxy-glycero-3-phosphate, choline |

Of the reaction products, particularly lysophosphatidic acid (LPA) and sphingosine-1-phosphate are biologically important substances because LPA has physiological activities such as cell growth promotion, smooth muscle contraction, promotion of tumor cell invasion and the like, and sphingosine-1-phosphate has physiological activities such as chemotactic kinetic regulation, growth of tumor cell and the like.

In addition, it is possible for those of ordinary skill in the art to prepare an antibody according to a conventional method using a purified LPLD or recombinant LPLD as an immune antigen. Alternatively, a peptide antibody corresponding to a partial amino acid sequence can be prepared based on the amino acid sequence depicted in SEQ ID NO:2.

An immune antigen is preferably prepared by mixing with an adjuvant. When the antigen has low immunity, the antigen is preferably conjugated with a carrier protein (e.g., bovine serum albumin (BSA) etc.) and used. The animal used for immunization target is, for example, mammals such as horse, sheep, goat, rabbit, guinea pig, mouse and the like. For immunization, these mammals are given an intrasubcutaneous, intramuscular, intravenous, intra-foot pad or intraperitoneal injection once to several times. In general, 1 to 4 times of immunizations are done about every 1-2 weeks from the first immunization, and after about 1-4 weeks, the final immunization is done. Several days after the final immunization, a specific antibody is obtained from the immunized animal as an anti-serum. It is also possible to prepare a monoclonal antibody by immunizing mouse, guinea pig and the like by a conventionally known method. The antibody is preferably purified as necessary before use. To be specific, the purification method is exemplified by an ammonium sulfate precipitation method, a method using protein A, and a method using an affinity column conjugated with an antigen polypeptide and/or peptide and the like.

The obtained specific antibody is useful as a tool for further purification of LPLD and investigation of LPLD and various diseases and phenomena considered to involve LPLD.

The lysophospholipid used as a substrate for the LPLD activity assay method of the present invention and a screening method of a substance inhibiting LPLD activity of the present invention is represented by the following formula (I) 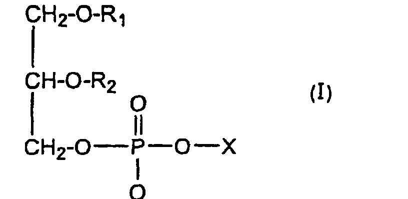 wherein R₁ and R₂ are different from each other and one of them is a saturated or unsaturated fatty acid residue having 8 to 22 carbon atoms, and the other is a hydrogen atom or an acetyl group, and X is a chromophore.

In the present invention, the "saturated or unsaturated fatty acid residue having 8 to 22 carbon atoms" is exemplified by residues of saturated fatty acid such as caprylic acid, capric acid, lauric acid, myristic acid, palmitic acid, stearic acid, arachidic acid, behenic acid and the like, and residues of unsaturated fatty acid such as spermic acid, palmitoleic acid, oleic acid, vaccenic acid, linoleic acid, α-linolenic acid, γ-linolenic acid, α-eleostearic acid, arachidonic acid, eicosapentaenoic acid, docosahexaenoic acid and the like, and the like, with preference give to a linoleic acid residue, an oleic acid residue, a palmitic acid residue and a myristic acid residue.

In the formula (I), the "chromophore" for X is a residue that can be a substance that absorbs visible light or UV rays or a substance that emits fluorescence (hereinafter these substances are to be also referred to as a chromogenic compound), upon release from the lysophosphblipid, or a precursor of the substance. As used herein, the precursor is one that can be a substance that absorbs visible light or UV rays or a substance that emits fluorescence, upon reaction with a different chromogenic reagent.

A specific chromophore is, for example, a residue containing a substituted phenol moiety [e.g., p-nitrophenol, 5-hydroxy-2-nitrobenzoic acid, 2-hydroxy-5-benzoic acid, N,N,N-trimethyl-N-(5-hydroxy-2-nitrophenyl)ammonium chloride, N,N,N-trimethyl-N-(2-hydroxy-5-nitrophenyl)ammonium chloride, 3-amino-4-nitrophenol, 2-amino-4-nitrophenol, 5-hydroxy-2-nitrobenzenesulfonic acid, o-nitrophenol etc.], a residue containing a naphthol or naphthol moiety [e.g., α-naphthol, 4-amino-1-naphthol, 4-hydroxy-1-trimethylammonium naphthalene chloride, 4-hydroxy-1-naphthoic acid, 3-amino-1-naphthol, 4-hydroxy-2-trimethylammoniumnaphthalene chloride, 4-hydroxy-2-naphthoic acid, 8-amino-1-naphthol, 8-hydroxy-1-trimethylammoniumnaphthalene chloride, 8-hydroxy-1-naphthoic acid, 5-amino-1-naphthol, 5-hydroxy-1-trimethylammoniumnaphthalene chloride, 5-hydroxy-1-naphthoic acid and 2-nitro-1-naphthol etc.], a residue containing a substituted umbelliferone moiety [e.g., 4-methylumbelliferone, 3-amino-4-methylumbelliferone, 3-trimethylammonium-4-methylumbelliferone chloride, 4-methyl-5-aminoumbelliferone, 4-methyl-6-aminoumbelliferone, 5-trimethylammonium-4-methylumbelliferone chloride, 6-trimethylammonium-4-methylumbelliferone chloride, 4-methylumbelliferone-5-sulfonic acid, 4-methylumbelliferone-6-sulfonic acid, 3-trimethylammonium-4-methylumbelliferone-5-sulfonic acid ester, 3-trimethylammonium-4-methylumbelliferone-5-carboxylic acid chloride and 3-trimethylammonium-4-methylumbelliferone chloride etc.], and the like.

The residue containing a substituted phenol moiety is preferably a p-nitrophenyl group, and by enzymolysis of lysophospholipid of the present invention, to which a p-nitrophenyl-group is bonded, a chromogenic compound, p-nitrophenol, is released. The p-nitrophenol shows the maximum absorption at around 400 nm (preferably 405 nm).

The residue containing a naphthol or naphthol moiety is preferably an α-naphthyl group, and by enzymolysis of lysophospholipid of the present invention, to which an α-naphthyl group is bonded, a precursor of a chromogenic compound, α-naphthol, is released. The α-naphthol can be a chromogenic compound by diazotization.

The residue containing a substituted umbelliferone moiety is preferably a 4-methylumbelliferyl group, and by enzymolysis of lysophospholipid of the present invention, to which a 4-methylumbelliferyl group is bonded, a chromogenic compound, 4-methylumbelliferone, is released. 4-Methylumbelliferone is a fluorescent compound and the fluorescence can be quantitatively determined by a spectrofluoro-photometer and the like.

In view of the easiness of the assay, p-nitrophenyl group and α-naphthyl group are preferable as chromophore.

The lysophospholipid of the present invention, which is represented by the formula (I), can be preferably produced by suitably combining known methods. While the detail will be explained in Synthetic Examples below, for example, a commercially available phospholipid is used as a starting material, which is subjected to enzymolysis with phospholipase C, and the obtained diglyceride and a commercially available compound having a chromophore are reacted in a solvent in the presence of a condensation agent to give a phospholipid having a chromophore bonded thereto (as a synthesis method of the phospholipid, a method analogous to the method described in Analytica. Chimica. Acta. 304, 249-254, 1995 can be also used). The phospholipid is subjected to enzymolysis by phospholipase A or lipase to give lysophospholipid of the present invention. To be specific, the following method is exemplified.

A commercially available soybean-derived phosphatidylcholine or yolk-derived phosphatidylcholine is used as a starting material, which is subjected to enzymolysis with phospholipase C (e.g., Welch bacillus-derived), and the obtained soybean-derived or yolk-derived diglyceride and commercially available 4-nitrophenyl phosphoro dichloride are reacted with a condensation agent, such as triisopropylbenzenesulfonyl chloride and the like, in a solvent such as anhydrous pyridine and the like to give a soybean-derived or yolk-derived phosphatidyl p-nitrophenol. By enzymolysis of the thus-obtained compound by phospholipase A2 (e.g., cobra venom-derived), a novel soybean-derived or yolk-derived 1-acylphosphatidyl-p-nitrophenol of the present invention, and by enzymolysis by lipase (e.g., Rhizopus stolonifer-derived), a novel soybean-derived or yolk-derived 2-acylphosphatidyl-p-nitrophenol of the present invention can be obtained.

As a different method, a commercially available soybean-derived phosphatidylcholine is used as a starting material, which is subjected to enzymolysis by phospholipase A2 or lipase, and the obtained soybean-derived 1-acylphosphatidylcholine or soybean-derived 2-acylphosphatidylcholine is reacted with acetic anhydride in a solvent such as chloroform and the like in the presence of a catalyst such as 4-dimethylaminopyridine, N,N-diisopropylamine and the like to give soybean-derived 1-acyl-2-acetyl-diglyceride or soybean-derived 1-acetyl-2-acyl-diglyceride. A p-nitrophenyl group is introduced into these compounds by a method similar to the aforementioned method, whereby novel soybean-derived 1-acyl-2-acetyl-phosphatidyl-p-nitrophenol or soybean-derived 1-acetyl-2-acyl-phosphatidyl-p-nitrophenol of the present invention is obtained.

The present invention provides a reagent for an LPLD activity assay, which contains the above-mentioned lysophospholipid as an active ingredient. The reagent for the assay can be used for the screening method of a substance that inhibits LPLD activity to be mentioned below for the assay of the activity. The reagent for assay may contain, in addition to lysophospholipid as a substrate, additives such as excipient, buffer, stabilizer, preservative and the like, and where necessary, an enzyme activator and an agent for the relaxation of an influence of contaminant substances may be added. The form of the reagent for assay of the present invention is not particularly limited and may be a dry powder, liquid and_the like. When a dry powder is formed,_where a treatment of dissolution, dilution and the like is necessary when in use, the elements necessary for the treatment (e.g., buffer and the like) may be included in a kit for an activity assay of LPLD of the present invention or a screening kit of a substance that inhibits LPLD activity of the present invention. A solution necessary for a treatment such as dissolution, dilution and the like is determined according to the solubility of the substrate, and it may be of an aqueous type such as a buffer and the like, or of an organic solvent type. Like the combination of the first reaction reagent and the second reaction reagent, moreover, two or more kinds of the reagents allowing various timings of addition to the reaction mixture may be appropriately combined, or one reagent system may be employed.

The present invention provides a method for assaying an LPLD activity, which is characterized by the use of the aforementioned lysophospholipid having chromophore as a substrate, and a screening method of a substance that inhibits LPLD activity. The assay method and the screening method commonly include the following steps.
(1) Lysophospholipid of the formula (I) (mentioned above) is added to a sample to react the lysophospholipid with LPLD in a sample.
(2) The amount of the chromogenic compound released by the above-mentioned step (1) is colorimetrically quantitatively determined,
provided that, when the chromogenic compound is released as a precursor thereof, it is converted to a chromogenic compound using a suitable chromogenic reagent, and the amount of the compound is measured by colorimetric quantitative determination.

In the screening method of a substance that inhibits LPLD activity of the present invention, a step of adding a test substance to a sample containing the LPLD and a step of comparing the amounts of a chromogenic compound released when a test substance is added and that without the addition are included, in addition to the above-mentioned step. This comparison step is performed by colorimetric quantitative determination (mentioned later) of the level of color development of each case.

The "test substance" intends any object whose influence on the LPLD activity is to be examined, which may be known or new. It may be a synthesized product or a naturally occurring product. The mode thereof is not particularly limited as long as it does not inhibit the reaction between LPLD and lysophospholipid, and may be purified as a compound or an unpurified composition, and solid or liquid. In the case of a liquid, the solvent thereof is not particularly limited.

The "sample" intends any object whose inclusion or non-inclusion of LPLD and whose level of activity are to be assayed, including various biological samples possibly containing LPLD, standard samples for the assay, control samples for the assay and various buffers free of LPLD. More specifically, for example, body fluid components used for clinical testing such as whole blood, serum, plasma, urine, saliva, ascites, spinal fluid and the like, those used for basic studies such as animal and plant cell extract solutions and the like are mentioned.

As the "sample containing the LPLD" which is used for the screening method of the present invention, those similar to the above-mentioned "sample" are used. A sample containing a known amount of LPLD can be subjected to the screening method of the present invention. In addition, the same samples containing LPLD can be also used for the screening method of the present invention, because, in the present invention, screening is performed by comparing the level of activity between addition and no addition of a test substance, which obliterates the need to particularly determine the absolute amount of LPLD contained therein.

Preferably, as the "sample containing LPLD", a sample containing a known amount of highly purified LPLD, more preferably a sample containing a known amount of recombinant LPLD, is used. Particularly, when the sample is a serum, a serum containing a known amount of highly purified LPLD or a serum containing a known amount of recombinant LPLD can be used as a useful control serum as a control sample or a standard sample for the activity assay method and the screening method of the present invention.

A sample containing a known amount of highly purified LPLD can be obtained by subjecting a sample containing the LPLD to a purification process, as mentioned above. Detailed step is described later in Examples. Alternatively, it can be prepared by adding a known amount of highly purified LPLD to an LPLD-free sample or a sample whose LPLD content is known.

A sample containing a known amount of recombinant LPLD can be obtained by adding a known amount of recombinant LPLD (the aforementioned) to a sample.

While the reaction conditions of a substrate, lysophospholipid, and a sample are appropriately determined depending on the kind and condition of the substrate and sample to be used, they are generally as follows.

### Reaction temperature

The temperature is not particularly limited as long as the substrate is stable and can maintain the LPLD activity. In consideration of the easiness, automation and the like of the assay, the assay is conducted generally at 25-40°C, preferably about 35-40°C.

### Reaction time

The present invention includes colorimetric quantitative determination of the amount of a released chromogenic compound, which includes measurement of time course changes in absorbance. Accordingly, monitoring is started immediately after the start of the reaction of lysophospholipid with a sample. For release of a chromogenic compound from the substrate, the reaction is carried out in generally 5 min to several hours, though subject to change depending on the kind of the substrate and other reaction conditions. Accordingly, changes in absorbance are preferably monitored for 5 min to one hour in the present invention.

### Reaction pH

The pH is not particularly limited as long as LPLD reacts with the substrate and the enzyme reaction thereof can be assayed. Depending on the chromophore to be used, the pH for LPLD activity and that for chromogenic reaction may be different. For example, when a p-nitrophenyl group is used as a chromophore, p-nitrophenol released by the LPLD reaction develops color only under alkaline conditions (Saiji Kanashima et al., Journal of Medical Technology 41, 1025-1028, 1997). Therefore, when the optimal pH of LPLD activity is near acidic range or neutral range, a sample containing LPLD and the substrate are reacted for a given time, and chromogenic reaction can be carried out under alkaline conditions. When the optimal pH of LPLD activity is in the alkaline range, however, the LPLD reaction and the chromogenic reaction can be simultaneously carried out at the same pH.

### Substrate concentration

While the substrate concentration varies depending on the kind of the substrate and other reaction conditions, it is not particularly limited as long as it is not susceptible to an influence of contaminant components and the amount thereof permits sufficient reaction with LPLD in a sample, and a preferable amount thereof is determined. To be specific, the final concentration is not less than 1 mmol/L, preferably 2 mmol/L - 10 mmol/L, particularly preferably about 2 mmol/L - 5 mmol/L. When it is less than 1 mmol/L, the difference from the blank becomes small and a significant value is difficult to obtain.

The amount of the released chromogenic compound is measured by colorimetric quantitative determination. The method for the colorimetric quantitative determination is appropriately determined according to the kind of the chromogenic compound, and the measurement is carried out according to the method known in this field. For example, when the chromogenic compound is p-nitrophenol, the obtained yellow color developed is measured as changes in absorbance near 400 nm by a spectrophotometer and the like. When the chromogenic compound is 4-methylumbelliferone, its fluorescence is measured by a spectrofluoro-photometer and the like. In the case of α-naphthol, a diazotization agent is used to conduct a coupling reaction to give a chromogenic compound, or an azo compound, whose color development is measured by a spectrophotometer and the like. As the diazotization agent, for example, Fast Red TR, p-diazobenzenesulfonic acid and the like are mentioned. When an additional chromogenic reagent such as a diazotization agent and the like is necessary, the reagent may be contained in an LPLD activity assay kit and a screening kit of a substance that inhibits LPLD activity, of the present invention to be mentioned later.

In the screening method of the present invention, addition of a test substance and a substrate, lysophospholipid, of the present invention to a sample is appropriately determined to be conducted when the effect of the test substance on the LPLD activity is sufficiently reflected. For example, when the effect quickly appears, the test substance and the substrate, lysophospholipid, can be almost simultaneously added. Generally, however, the test substance and LPLD in the sample are sufficiently reacted in advance, and the substrate, lysophospholipid of the present invention, is added, to conduct the LPLD activity assay of the present invention.

In the present invention, respective components necessary for LPLD activity assay of the present invention or screening of a substance that inhibits LPLD activity of the present invention may be collectively packaged in a kit.

Where necessary, such kit contains, in addition to lysophospholipid to be the substrate, components necessary for the reaction such as a solution for dissolution, chromogenic reagent and the like. Preferably, a standard solution and a control solution are included. Furthermore, in addition to the components necessary for the assay, a vessel for reaction, an instruction manual and the like are preferably packaged together for convenience. A screening kit of a substance that inhibits LPLD activity preferably contains a sample containing the LPLD. The "sample containing the LPLD" is preferably "a sample containing a known amount of highly purified LPLD", and particularly preferably "a sample containing a sample containing a known amount of recombinant LPLD". By including a sample containing LPLD in the kit in advance, changes in the LPLD activity with or without addition of a test substance can be assayed using the same sample.

A substance that inhibits LPLD activity, which is obtained by the screening method of the present invention, can suppress, by the LPLD inhibitory action, the production of LPA and can be preferably used for various diseases considered to be caused by excess or abnormal production of LPA. For example, it can be used for the prophylaxis or treatment of arteriosclerosis.

### Examples

While the present invention is explained in detail in the following by referring to Examples, the present invention is not limited by these examples in any way. Unless particularly indicated, respective materials to be used in the following Examples, such as reagent and the like, are commercially available and can be used according to the attached instruction manual.

### Example 1: synthesis of 1-acyl-lysophosphatidyl-p-nitrophenol-(1)

### soybean-derived 1-linoleoyl-2-hydroxy-phosphatidyl-p-nitrophenol:

### (1) Synthesis of soybean-derived 1,2-dilinoleoyl-phosphatidyl-p-nitrophenol

Soybean-derived dilinoleoylglycerol (2.68 g, 4.3 mmol) and 4-nitrophenylphosphoro dichloride (0.916 g, 3.6 mmol) were dried on P₂O₅ for about 2 hr and anhydrous pyridine (10 mL) was added. After thorough suspension, 2,4,6-triisopropylbenzenesulfonyl chloride (2.71 g, 8.95 mmol) was added and the mixture was reacted at room temperature overnight. Water was poured into the reaction mixture, and the mixture was mixed and stirred, and ethanol and toluene were added, which was followed by concentration under reduced pressure to dryness. Diethyl ether was added to the obtained dry product and an insoluble portion was removed to give a soluble portion, which was dissolved in a small amount of chloroform and applied to silica gel column chromatography activated with the same type of solvent for purification using a chloroform-methanol type eluent to give 1.45 g of a yellow oil.

### (2) Soybean-derived 1-linoleoyl-2-hydroxy-phosphatidyl-p-nitrophenol

The yellow oil (564 mg) obtained in the above-mentioned (1) was dissolved in diethyl ether (20 mL) and phospholipase A2 (100 mg, cobra venom-derived; Wako Pure Chemical Industries, Ltd.) was dissolved in 0.1 mol/L borate-phosphate buffer (10 mL, pH 6.5) and 1 mol/L calcium chloride (0.1 mL). They were mixed and reacted for 48 h. After the reaction, a diethyl ether fraction was concentrated under reduced pressure and the concentrate was applied to silica gel column chromatography for purification using a chloroform-methanol type eluent to give 252 mg of the title compound as a yellow oil.
ESI-MS (Negative) m/z 554 [m-H]⁻
NMR (solvent CDCl₃)
¹H-NMR: 0.87-0.92 (m, 3H), 1.27-1.48 (m, 20H), 2.05-2.11(m, 4H), 2.76-2.79 (m, 2H), 3.55-4.07 (m, 4H), 4.53 (s, 1H), 5.33-5,41 (m, 2H), 7.32-7.35 (d, 2H), 8.06-8.09 (d, 2H) ¹³C-NMR: 13.52 (methyl.) , 22.04-33.42 (methylenes, 10 signals), 62.05 (methylenes), 74.96 (methine), 119.58 and 124.89 (aroma), 127.36, 127.65, 129.37 and 129.74 (vinyl C), 142.98 and 156.94 (aroma), 173.54 (C=0)

### Example 2: synthesis of 1-acyl-lysophosphatidyl-p-nitrophenol-(2)

### yolk-derived 1-oleoyl-2-hydroxy-phosphatidyl-p-nitrophenol:

(1) Synthesis of yolk-derived diacylphosphatidyl-p-nitrophenol
   By the synthesis and purification according to Example 1 (1) using yolk-derived diacylglycerol (1.75 g, 2.95 mmol), 4-nitrophenylphosphoro dichloride (0.686 g, 2.68 mmol) and'2,4,6-triisopropylbenzenesulfonyl chloride (2.02 g, 6.7 mmol), a yellow oil (1.41 g) was obtained.
(2) Yolk-derived 1-oleoyl-2-hydroxy-phosphatidyl-p-nitrophenol
   By the hydrolysis and purification according to Example 1 (2) using the yellow oil (573 mg) obtained in the above-mentioned (1) and phospholipase A2 (123 mg, cobra venom-derived; Wako Pure Chemical Industries, Ltd.), the title compound (221 mg) was obtained as a yellow oil.
   ESI-MS (Negative) m/z 556 [m-H]⁻
   NMR (solvent CDCl₃)
   ¹H-NMR: 0.87-0.91 (m, 3H), 1.28-1.48 (m, 22H), 2.02-2.11 (m, 4H) , 2.78-2.79 (m, 2H) , 3.55-4.06 (m, 4H), 4.52 (s, 1H), 5.33-5.36 (m, 2H), 7.32-7.35 (d, 2H), 8.07-8.10 (d, 2H) ¹³C-NMR: 13.56 (methyl), 22.15-33.43 (methylenes, 10 signals), 62.40 (methylenes), 75.02 (methine), 119.59 and 124.91 (aroma), 127.64, 129.07 and 129.57 (vinyl C), 143.00 and 157.03 (aroma), 173.55 (C=O)

### Example 3: synthesis of 2-acyl-lysophosphatidyl-p-nitrophenol-(1)

### Soybean-derived 1-hydroxy-2-linoleoyl-phosphatidyl-p-nitrophenol:

The compound (1.45 g) obtained in the above-mentioned Example 1 (1) was dissolved in diethyl ether (50 mL), and lipase (1 g, Rhizopus stolonifer-derived: SEIKAGAKU CORPORATION) was dissolved in 0.1 mol/L borate-phosphate buffer (14 mL, pH 6.5) and 1 mol/L calcium chloride (0.1 mL). They were mixed and reacted for 48 h. After the reaction, a diethyl ether fraction was concentrated under reduced pressure and the concentrate was applied to silica gel column chromatography for purification using a chloroform-methanol type eluent to give 568 mg of the title compound as a yellow oil.
ESI-MS (Negative) m/z 554 [m-H]⁻
NMR (solvent CDCl₃)
¹H-NMR: 0.87-0.92 (m, 3H), 1.23-1.47 (m, 20H) , 2.04-2.17 (m, 4H), 2.75-2.79 (m, 2H), 3.62-4.12 (m, 4H), 4.96-4.98 (m, 1H), 5.32-5.41 (m, 2H), 7.27-7.31 (d, 2H), 8.01-8.04 (d, 2H) ¹³C-NMR: 13.52 (methyl), 22.04-33.66 (methylenes, 10 signals), 59.74 and 64.31(methylenes), 72.12 (methylene), 119.50 and 124.86 (aroma), 127.36, 127.64, 129.37 and 129.74 (vinyl C), 142.94 and 156.83 (aroma), 173.40 (C=O)

### Example 4: synthesis of 2-acyl-lysophosphatidyl-p-nitrophenol-(2)

### Yolk-derived 1-hydroxy-2-oleoyl-phosphatidyl-p-nitrophenol:

By the hydrolysis and purification according to Example 3 using the compound (714 mg) obtained in the above-mentioned Example 2(1) and lipase (700 mg), the title compound (374 mg) was obtained.
ESI-MS (Negative) m/z 556 [m-H]⁻
NMR (solvent CDCl₃)
¹H-NMR: 0.87-0.91 (m, 3H), 1.24-1.47 (m, 22H), 2.02-2.17 (m, 4H) , 2.77-2.83 (m, 2H) , 3.63-4.12 (m, 4H), 4.97 (s, 1H), 5.36 (m, 2H), 7.32-7.31 (d, 2H), 8.02-8. 04 (d, 2H)
¹³C-NMR: 13.55 (methyl), 22.14-33.66 (methylenes, 11 signals), 59.77 and 64.32 (methylenes), 72.05 (methylene), 119.57 and 124.88 (aroma), 127.35, 127.65, 129.04 and 129.57 (vinyl C), 143.02 and 156.79 (aroma), 173.43 (C=O)

### Example 5: activity assay 1 (reaction pH)

### (1) Preparation of reagent

Reagent A: N-(2-hydroxyethyl)piperazine-N'-3-propanesulfonic acid (200 mmol/L), Triton X-100 (0.02%)
Reagent B: reagent A was separated and adjusted to pH 7.6, 7.8, 8.0, 8.2, 8.4 and 8.6, diluted 2-fold to give reagent B having various pHs.
Reagent C: pH 4.5
citric acid monohydrate (10 mmol/L), Triton X-100 (0.01%), 1-hydroxy-2-linoleoyl-phosphatidyl-p-nitrophenol (Example 3; 10 mmol/L) prepared in the above-mentioned Example.

### (2) Sample

Two kinds of commercially available control serum, respective test sample 1 (CONSERA: Nissui Pharmaceutical Co.) and test sample 2 (Lipid Serum II: Eiken Chemical Co., Ltd.), and 5% human serum albumin (fatty acid free: Sigma) solution for determination of the activity of albumin fraction in the serum, were used.

### (3) Assay

Each sample (10 µL) and reagent B (150 µL) were dispensed to a microplate and the plate was incubated at 37°C for about 5 min. Thereto was added reagent C (50 µL) and the absorbance at a dominant wavelength of 405 nm and sub wavelength of 540 nm was measured with the lapse of time. The LPLD activity was determined based on changes in absorbance per minute that depend on p-nitrophenol released by the action of LPLD. The activity is expressed by the amount of p-nitrophenol released in one minute per 1 mL of the reaction mixture. The results are shown in Fig. 1. While the activity is potent when pH is on the alkaline side, the activity in a human serum albumin fraction also increases. At pH 8 or below, therefore, an activity assay is possible where an influence of albumin is less.

### Example 6: activity assay 2 (substrate)

### (1) Preparation of reagent

Reagent A: pH 8.0
   2-[4- (2-hydroxyethyl) -1-piperazinyl)ethanesulfonic acid (100 mmol/L), Triton X-100 (0.01%)
Reagent B: pH 4.5
   citric acid monohydrate (10 mmol/L), Triton X-100 (0.01%) reagent C: solutions corresponding to 100 mmol/L of soybean-derived 1-hydroxy-2-linoleoyl-phosphatidyl-p-nitrophenol (Example 3; substrate A), soybean-derived 1-linoleoyl-2-hydroxy-phosphatidyl-p-nitrophenol (Example 1; substrate B), yolk-derived 1-hydroxy-2-oleoyl-phosphatidyl-p-nitrophenol (Example 4; substrate C), and yolk-derived 1-oleoyl-2-hydroxy-phosphatidyl-p-nitrophenol (Example 2; substrate D), prepared in the above-mentioned Examples, were diluted 10-fold with reagent B and used as reagent C.

### (2) Sample

Using human serum as a sample, 5% human serum albumin (fatty acid free: Sigma) solution was used for determining the activity in albumin fraction in the serum, and a 5 U/mL solution of Streptomyces-derived PLD (product abbreviation PLDP: Asahi Chemical Industry Co., Ltd.) was used for determining the reactivity with phospholipase D (PLD).

### (3) Assay

Each sample (10 µL) and reagent A (150 µL) were dispensed to a microplate and the plate was incubated at 37°C for about 5 min. Thereto was added reagent C (50 µL) and the absorbance at a dominant wavelength of 405 nm and sub wavelength of 540 nm was measured with the lapse of time. The LPLD activity was determined based on changes in absorbance per minute that depend on p-nitrophenol released by the action of LPLD. The activity is expressed by the amount of p-nitrophenol released in one minute per 1 mL of the reaction mixture. The results are shown in Fig. 2. The response to human serum albumin was higher for 1-acyl-2-hydroxy-phosphatidyl-p-nitrophenol such as substrates B and D than for 1-hydroxy-2-acyl-phosphatidyl-p-nitrophenol such as substrates A and C, but the response to human serum was higher for 1-hydroxy-2-acyl-phosphatidyl-p-nitrophenol than for 1-acyl-2-hydroxy-phosphatidyl-p-nitrophenol. This suggests that, particularly when 1-hydroxy-2-acyl-phosphatidyl-p-nitrophenol is used as a substrate, the LPLD activity can be assayed with a smaller influence of albumin.

It has been found that, while these substrates show various levels of reaction, all can be a substrate for Streptomyces-derived PLD. While there is no report on the presence of PLD in normal human serum, when PLD is coexistent in a sample, the use of substrate A or substrate B from among the above-mentioned substrates is preferable.

### Example 7: activity assay 3 (substrate concentration)

### (1) Preparation of reagent

Reagent A: pH 8.0
   2-[4-(2-hydroxyethyl)-1-piperazinyl]ethanesulfonic acid (100 mmol/L), Triton X-100 (0.01%)
Reagent B: pH 4 . 5
   citric acid monohydrate (10 mmol/L), Triton X-100 (0.01%) reagent C: The soybean-derived 1-hydroxy-2-linoleoyl-phosphatidyl-p-nitrophenol (Example 3) prepared in the above-mentioned Examples as a substrate was diluted with reagent B to 0, 0.5, 1.0, 2.0, 3.0, 5.0, 10, 20, 30 and 50 mmol/L and used as reagent C.

### (2) Sample

Using human serum as a sample, 5% human serum albumin (fatty acid free: Sigma) solution was used for determining the reaction of albumin in the serum.

### (3) Assay

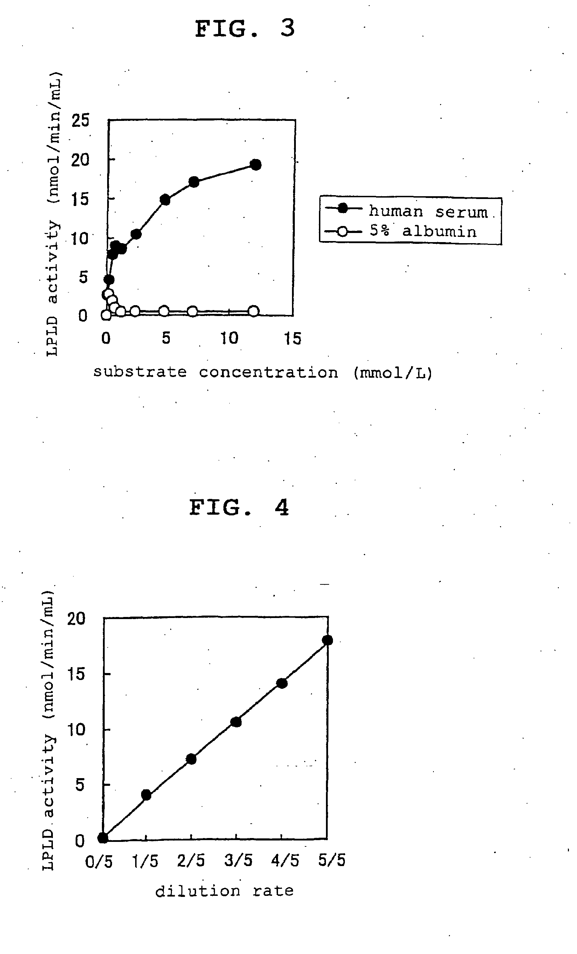

Each sample (10 µL) and reagent A (150 µL) were dispensed to a microplate and the plate was incubated at 37°C for about 5 min. Thereto was added reagent C (50 µL) and the absorbance at a dominant wavelength of 405 nm and sub wavelength of 540 nm was measured with the lapse of time. The LPLD activity was determined based on changes in absorbance per minute that depend on p-nitrophenol released by the action of LPLD. The activity is expressed by the amount of p-nitrophenol released in one minute per 1 mL of the reaction mixture. The results are shown in Fig. 3. As a final concentration of the substrate, addition of not less than 5 mmol/L is desirable.

### Example 8: activity assay 4 (dilution linearity of sample)

### (1) Preparation of reagent

Reagent A: pH 8.0
   2-[4-(2-hydroxyethyl) -1-piperazinyl]ethanesulfonic acid (100 mmol/L), Triton X-100 (0.01%)
Reagent B: pH 4.5
   citric acid monohydrate (10 mmol/L), Triton X-100 (0.01%), soybean-derived 1-hydroxy-2-linoleoyl-phosphatidyl-p-nitrophenol (Example 3; 20 mmol/L)

### (2) Sample

As a sample, 5 serial dilutions of human serum with physiological brine were used.

### (3) Assay

Each sample (10 µL) and reagent A (150 µL) were dispensed to a microplate and the plate was incubated at 37°C for about 5 min. Thereto was added reagent B (50 µL) and the absorbance at a dominant wavelength of 405 nm and sub wavelength of 540 nm was measured with the lapse of time. The LPLD activity was determined based on changes in absorbance per minute that depend on p-nitrophenol released by the action of LPLD. The activity is expressed by the amount of p-nitrophenol released in one minute per 1 mL of the reaction mixture. The results are shown in Fig. 4. As shown in Fig. 4, fine linearity with the passage through the origin was obtained. This suggests that the activity assay method of the present invention affords quantitative determination of LPLD activity in human serum.

### Example 9: purification of lysophospholipase D (bovine lysophospholipase D)

All the following steps were performed at 4°C except elution of (3).

### (1) Precipitation by polyethylene glycol (PEG)

50% PEG was gradually added to the starting material, fetal calf serum (FCS, 2 L), to the PEG final concentration of 5%. After stirring for 10 min, the reaction mixture was centrifuged at 20,400 g for 60 min and the supernatant was recovered. 50% PEG was gradually added to the final concentration of 10% and the reaction mixture was centrifuged at 20,400 g for 60 min. The obtained precipitate was suspended in buffer A (20 mM Tris-HCl, 400 mL, pH 7.4).

### (2) Affinity chromatography using blue sepharose

The aforementioned suspension was added to blue sepharose 6FF (Pharmacia Biotech, column size 50 mL) equilibrated with buffer A, and washed with buffer A. LPLD was eluted by gravity-drop by linear concentration gradient (0-2 M) of NaCl in Buffer A and the fractions showing the LPLD activity were collected.

### (3) Affinity chromatography using concanavalin A (ConA) sepharose

The aforementioned activity fractions were added to ConA sepharose (Pharmacia Biotech, column size, 3 mL) equilibrated with buffer B (buffer A containing 500 mM NaCl), and washed with buffer B. LPLD was eluted by gravity-drop using buffer C (buffer B containing 5 mM of α-methylmannoside) at room temperature and the fractions showing an LPLD activity were collected and dialyzed against buffer A.

### (4) Anion exchange chromatography using BioAssistQ column

The aforementioned dialysate was added to BioAssistQ (Tosoh Corporation, column size 0.8 mL) equilibrated with buffer D (20 mM Tris-HCl, pH 8.0)) and washed with buffer D. LPLD was eluted by linear concentration gradient (0-2 M) of NaCl in Buffer D and the fractions showing the LPLD activity were collected.

### (5) Affinity chromatography using heparin column

The aforementioned active fractions were diluted 10-fold with buffer E (buffer A containing 10% glycerol) and added to HiTrap Heparin column (Pharmacia Biotech, column size 1 mL) equilibrated with buffer E, and washed with buffer E. LPLD was eluted at 0.5 mL/min by linear concentration gradient (0-2 M) of NaCl in Buffer E and the fractions showing the LPLD activity were collected.

### (6) Hydrophobic chromatography using RESOURCE PHE column

5M NaCl was added to the aforementioned active fraction to make the NaCl final concentration 2M. This was added to RESOURCE PHE (Pharmacia Biotech, column size 1 mL) equilibrated with buffer F (buffer A containing 2M NaCl), and washed with buffer F. LPLD was eluted with 1 mM EDTA at 1.0 mL/min and the pass fractions showing the LPLD activity were collected.

### (7) Non-specific adsorption using hydroxyapatite (HAT)

The aforementioned activity fractions were diluted 10-fold with buffer G (1 mM NaH₂PO₄-NaOH containing 10% glycerol, pH 7.4) and added to HAT (Bio-Rad, column size 1 ml) equilibrated with buffer G, and washed with buffer G. LPLD was eluted at 0.8 mL/min by linear concentration gradient (1-150 mM) of NaH₂PO₄-NaOH in Buffer G and the fractions showing the LPLD activity were collected to give a partially purified product of bovine phospholipase D.

### (8) Assay of lysophospholipase D activity of each fraction

The starting material, FCS, and respective fractions obtained in the above-mentioned (1)-(7) were assayed for lysophospholipase D activity to confirm level of purification.

The lysophospholipase D activity of each fraction was determined in a reaction mixture (100 mM Tris hydrochloric acid (pH 9.0), 500 mM sodium chloride, 5 mM magnesium chloride, 0.05% Triton X-100, 1 mM lysophosphatidylcholine). A sample (FCS, either fraction obtained in (1)-(7)) was added to the aforementioned reaction mixture to 100 µL and the mixture was incubated at 37°C for 60 min. 15 µL therefrom was reacted with 200 µL of an assay reagent (100 mM Tris-hydrochloric acid (pH 8.0), 0.375 mM N-ethyl-N-(2-hydroxy-3-sulfopropyl)-3-methoxyaniline, 0.25 mM 4-aminoantipyrine, 0.01% Triton X-100, 7.5 U/mL peroxidase, 2.5 U/mL choline oxidase) for 10 min and the absorbance at 550 nm was measured. The measure was converted based on the absorbance obtained when 50 µM choline chloride was used instead of the aforementioned reaction mixture.

The results are shown in Table 2 as a purification table.

**Table 2**

| Purification step | Total activity | Protein | Specific activity | Purification fold | Yield |
|---|---|---|---|---|---|
| | (nmol/min) | (mg) | (nmol/min/mg) | | (%) |
| FCS | 7921.7 | 68300 | 0.116 | 1 | 100 |
| PEG precipitation | 1132.1 | 3720 | 0.3 | 2.6 | 14.3 |
| Blue sepharose | 1184.1. | 310.8 | 3.8 | 32.8 | 14.9 |
| ConA | 803.8 | 40.3 | 19.9 | 171.6 | 10.4 |
| BioAssistQ | 297.5 | 9.25 | 32.2 | 277.6 | 3.8 |
| heparin | 151.0 | 3.6 | 41.9 | 361.6 | 1.9 |
| RESOURSE | 134.3 | 0.2 | 371.5 | 3202.6 | 1.7 |
| HAT | 34.1 | 0.0668 | 510.5 | 4400.9 | 0.43 |

### Example 10: determination of partial amino acid sequence of bovine lysophospholipase D

The protein in a partially purified product of bovine lysophospholipase D obtained in Example 9 was precipitated with 10% trichloroacetic acid to concentrate bovine lysophospholipase D, and the protein was fractionated by 10% acrylamide SDS-PAGE. After staining with CBB, a protein band having a molecular weight of about 100 Kda, which showed good correlation with lysophospholipase D activity, was cut out from an acrylamide gel and subjected to a lysyl endopeptidase enzyme treatment. The obtained peptide fragments were fractionated by C4 reverse column chromatography. Of these peptides, the amino acid sequences of 3 peptides were determined by the Edman method.

(1) MHTARVRDIEHLTSLDFFRK (SEQ ID NO: 3)

(2) YASERNGVNVISGPIFDYDYDGLHDTEDK (SEQ ID NO: 4)

(3) FNHRWWGGQPLWITATK (SEQ ID NO: 5)

The obtained partial amino acid sequences were applied to a homology search using a BLAST-T program. As a result, all the obtained sequences showed an extremely high homology with autotaxin (SEQ ID NO: 2) of human, mouse and rat. The autotaxin is known to be a tumor cell motility-stimulating factor, and is a protein reported to have a nucleotide pyrophosphatase activity, phosphodiesterase activity and act on various nucleotide compounds, but no report has been documented that this protein and a protein analogous thereto have an LPLD activity and act on phospholipid.

### Example 11: expression of LPLD activity in recombinant host cell

Because an LPLD activity of autotaxin was expected from the results of Example 10, a transformant was prepared using cDNA of rat autotaxin and expression of LPLD activity by said transformant was examined.

From the reported sequence of rat autotaxin (SEQ ID NO: 1), a cDNA encoding rat autotaxin was isolated by polymerase chain reaction (RT-PCR) using reverse transcriptase. As a template, used was a cDNA synthesized from rat liver-derived RNA by reverse transcriptase.

The obtained cDNA was inserted into the BamH I/Xho I site of the expression vector pcDNA3 and myc tag was introduced into the C terminal of cDNA. The obtained cDNA was temporarily introduced into CHO-K1 cell using lipofectamine.

After 48 hours, the culture supernatant was recovered and expression was confirmed by western blot using an anti-myc tag antibody. In addition, expression of LPLD by the recombinant host cell was confirmed by the aforementioned LPLD activity assay method.

### Example 12: assay of LPLD activity using purified LPLD and the substrate of the present invention

(1) Synthesis of 1-myristoyl-phosphatidyl-p-nitrophenol
   (i) Synthesis of 1,2-dimyristoyl-phosphatidyl-p-nitrophenol
      Dimyristoylglycerol (2.02 g) and 4-nitrophenylphosphoro dichloride (1.00 g) were dried on P₂O₅ at room temperature for about 2 hr and anhydrous pyridine (12 mL) was added. After thorough suspension, 2,4,6-triisopropylbenzenesulfonyl chloride (1.13 g) was added and the mixture was reacted at room temperature overnight. Water was poured into the reaction mixture, and the mixture was mixed and stirred, and ethanol and toluene were added, which was followed by concentration under reduced pressure to dryness. Chloroform-methanol (2:1, 100 mL) was added to the obtained dry product, and after dissolution, water (20 mL) was added for partitioning. The lower layer was recovered. Water was removed with anhydrous sodium sulfate and the residue was filtrated. The filtrate was concentrated under reduced pressure to dryness. The obtained dry product was dissolved in a small amount of chloroform, dissolved therein and applied to silica gel column chromatography activated with the same type of solvent for purification using a chloroform-methanol-aqueous type eluent to give 1.4 g of a white powder.
   (ii) Synthesis of 1-myristoyl-phosphatidyl-p-nitrophenol

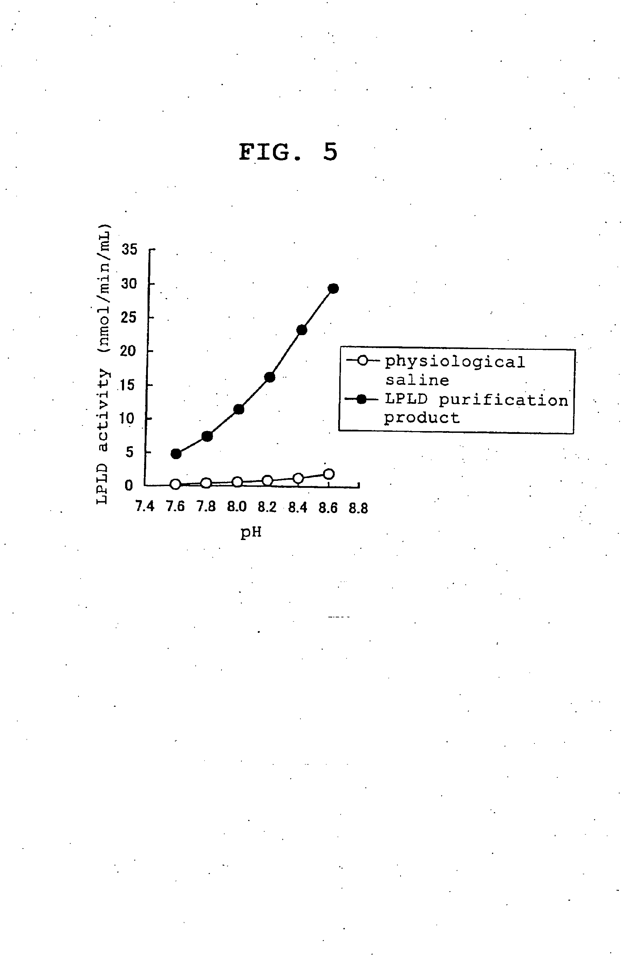

   The white powder (600 mg) obtained in the above-mentioned (i) was dissolved in chloroform (40 mL), then phospholipase A2 (Sigma, cobra venom-derived, 90 mg) was dissolved in 0.1 M borate-phosphate buffer (20 mL, pH 6.5) and 1M calcium chloride solution (0.1 mL). They were mixed and reacted at 35°C for 24 h. Chloroform-methanol (2:1, 100 mL) was added to the reaction mixture for partitioning, and the lower layer was recovered. Water was removed with anhydrous sodium sulfate and the residue was filtrated. The filtrate was concentrated under reduced pressure to dryness. The obtained dry product was applied to silica gel column chromatography for purification using a chloroform-methanol-aqueous type eluent to give the title compound (223 mg) as a white powder.
   ESI-MS (Negative) m/z 503[m-H]⁻
(2) Preparation of reagents
   reagent A: pH 8.0
      2-[4-(2-hydroxyethyl)-1-piperazinyl]ethanesulfonic acid(100 mmol/L), Triton X-100 (0.01%)
   Reagent B: pH 4.5
      citric acid monohydrate (10 mmol/L), Triton X-100 (0.01%) Reagent C: 1-myristoyl-phosphatidyl-p-nitrophenol was dissolved in reagent B to correspond to 10 mmol/L and used as reagent C.
(3) Assay
   For assay, biochemical automatic analyzer (Toshiba Corporation: TBA-80FR NEO) was used. The purified LPLD (8 µL) obtained in Example 9 and reagent A (240 µL) were pre-warmed at 37°C for about 15 min, and after addition of reagent C (80 µL), the absorbance at dominant wavelength 405 nm and sub wavelength of 505 nm was measured with lapse of time for about 15 min. The LPLD activity was determined based on changes in absorbance per minute that depends on p-nitrophenol released by the action of LPLD. The activity is expressed by the amount of p-nitrophenol released in one minute per 1 mL of the reaction mixture. The results are shown in Fig. 5. Similar to Fig._1 a stronger activity was found when pH was on the alkaline side.
   A similar assay is available when recombinant LPLD is used instead of purified LPLD.

### INDUSTRIAL APPLICABILITY

By the use of the novel lysophospholipid of the present invention as a substrate, a specific and high sensitivity LPLD activity assay becomes attainable. Moreover, by the use of a safe and conveniently detectable chromophore, a number of samples can be assayed rapidly. Furthermore, by the use of the novel lysophospholipid of the present invention as a substrate, a substance that inhibits LPLD activity can be screened.

This application is based on patent application Nos. 403530/2000 and 023784/2001 filed in Japan, the contents of which are hereby incorporated by reference.

## Claims

1. A lysophospholipid of the formula (I) 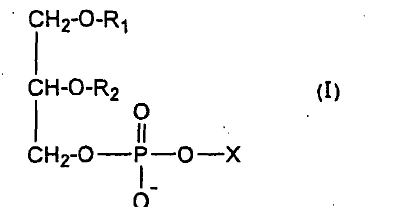 wherein R₁ and R₂ are different from each other and one of them is a saturated or unsaturated fatty acid residue having 8 to 22 carbon atoms, and the other is a hydrogen atom or an acetyl group, and X is a chromophore.

2. The lysophospholipid of claim 1, wherein the fatty acid residue is selected from the group consisting of a linoleic acid residue, an oleic acid residue, a palmitic acid residue and a myristic acid residue.

3. The lysophospholipid of claim 1 or 2, wherein the chromophore is a p-nitrophenyl group or an α-naphthyl group.

4. A reagent for assaying a lysophospholipase D activity, which comprises the lysophospholipid of claim 1 as an active ingredient.

5. A method of assaying a lysophospholipase D activity of a sample, comprising adding the lysophospholipid of claim 1 to the sample to allow reaction of the lysophospholipid and lysophospholipase D in the sample and colorimetrically quantitatively determining an amount of a chromogenic compound released by the reaction.

6. The assay method of claim 5, wherein the sample is selected from the group consisting of whole blood, serum, plasma, urine and saliva.

7. The assay method of claim 6, which is used for the diagnosis of at least one kind of disease selected from the group consisting of ovarian cancer, cervical cancer, endometrial cancer, breast cancer and an arteriosclerotic disease.

8. A kit for assay of a lysophospholipase D activity of a sample, comprising the lysophospholipid of claim 1.

9. The kit of claim 8, wherein the sample is selected from the group consisting of whole blood, serum, plasma, urine and saliva.

10. The kit of claim 8, which is used for the diagnosis of at least one kind of disease selected from the group consisting of ovarian cancer, cervical cancer, endometrial cancer, breast cancer and an arteriosclerotic disease.

11. A gene comprising a DNA of the following (a) or (b):
(a) a DNA encoding a protein having a lysophospholipase D activity, which consists of a base sequence depicted in SEQ ID No: 1,
(b) a DNA encoding a protein having a lysophospholipase D activity, which hybridizes with a DNA consisting of the base sequence of (a) under stringent conditions.

12. A protein of the following (a) or (b):
(a) a protein having a lysophospholipase D activity, which consists of an amino acid sequence depicted in SEQ ID No:2,
(b) a protein having a lysophospholipase D activity, which consists of an amino acid sequence of (a) wherein one to several amino acids have been deleted, substituted or added.

13. A recombinant vector comprising the gene of claim 11.

14. An expression vector functionally comprising the gene of claim 11.

15. A transformant obtained by transforming a host cell with the vector of claim 13 or 14.

16. A method of producing lysophospholipase D, which comprises culturing, under conditions that permit expression of a protein, a host cell transformed with the expression vector of claim 14 and harvesting a substance having a lysophospholipase D activity from the obtained culture.

17. A lysophospholipase D produced by the production method of claim 16.

18. The reagent for assaying a lysophospholipase D activity of claim 4, wherein the lysophospholipase D is a protein encoded by the gene of claim 11.

19. The reagent for assaying a lysophospholipase D activity of claim 4, wherein the lysophospholipase D is the protein of claim 12.

20. A control serum for a lysophospholipase D activity assay, which is obtained by adding a protein encoded by the gene of claim 11 to a serum.

21. A control serum for a lysophospholipase D activity assay, which is obtained by adding the protein of claim 12 to a serum.

22. A method for screening a substance that inhibits a lysophospholipase D activity, which comprises at least the following steps:
(i) a step for adding a test substance to a sample containing lysophospholipase D,
(ii) a step for adding the lysophospholipid of the above-mentioned (1) to a sample containing lysophospholipase D,
(iii) a step for colorimetrically quantitatively determining an amount of a chromogenic compound released by reacting the lysophospholipid and lysophospholipase D in a sample, and
(iv) a step for comparing the lysophospholipase D activity shown by the degree of color development with that when a test substance is not added.

23. The screening method of claim 22, wherein the lysophospholipase D is encoded by the gene of claim 11.

24. The screening method of claim 22, wherein the lysophospholipase D is the protein of claim 12.

25. A kit for screening a substance that inhibits a lysophospholipase D activity, which comprises the lysophospholipid of claim 1 and a sample containing lysophospholipase D.

26. The kit of claim 25, wherein the lysophospholipase D is a protein encoded by the gene of claim 11.

27. The kit of claim 25, wherein the lysophospholipase D is the protein of claim 12.


SEQUENCE LISTING
<110> AZWELL INC.

<120> Determination method of lisophospholipase D

<130> 09449

<150> JP 2000-403530
<151> 2000-12-28
<150> JP 2001-023784
<151> 2001-1-31

<160> 5

<170> PatentIn version 3.0

<210> 1

<211> 2696

<212> DNA

<213> Rattus norvegicus
<220>
<221> CDS
<222> (90)..(2675)

<400> 1
ggtacccaac agcctgaact cagagccccg agagcagagc attcagggca agcagaaaca 60

ccctgcagrk rcgagctcgg atctccacc atg gca aga caa ggc tgt ctc ggg 113
Met Ala Arg Gln Gly Cys Leu Gly
1               5

tca ttc cag gta ata tcc ttg ttc act ttt gcc atc agt gtc aat atc 161
Ser Phe Gln Val Ile Ser Leu Phe Thr Phe Ala Ile Ser Val Asn Ile
    10                  15                  20

tgc tta gga ttc aca gca agt cga att aag agg gca gaa tgg gat gaa 209
Cys Leu Gly Phe Thr Ala Ser Arg Ile Lys Arg Ala Glu Trp Asp Glu
25                  30                  35                  40

gga cct ccc aca gtg ctg tct gac tct cca tgg acc aac acc tct gga 257

Gly Pro Pro Thr Val Leu Ser Asp Ser Pro Trp Thr Asn Thr Ser Gly
                45                  50                  55

tcc tgc aaa ggt aga tgc ttt gag ctt caa gag gtt ggc cct cca gac 305
Ser Cys Lys Gly Arg Cys Phe Glu Leu Gln Glu Val Gly Pro Pro Asp
            60                  65                  70

tgt cgg tgt gac aac ctg tgt aag agc tac agc agc tgc tgc cac gat 353
Cys Arg Cys Asp Asn Leu Cys Lys Ser Tyr Ser Ser Cys Cys His Asp
        75                  80                  85

ttc gat gag ctc tgt ttg aaa aca gct cga ggc tgg gag tgc acc aaa 401
Phe Asp Glu Leu Cys Leu Lys Thr Ala Arg Gly Trp Glu Cys Thr Lys
    90                  95                  100

gac aga tgt ggg gaa gta cga aac gag gaa aat gcc tgt cac tgc tca 449
Asp Arg Cys Gly Glu Val Arg Asn Glu Glu Asn Ala Cys His Cys Ser
105                 110                 115                 120

gaa gac tgc ttg tcc agg gga gac tgc tgt acc aac tac caa gtg gtc 497
Glu Asp Cys Leu Ser Arg Gly Asp Cys Cys Thr Asn Tyr Gln Val Val
                125                 130                 135

tgc aaa gga gaa tca cac tgg gta gat gat gac tgc gaa gaa atc aaa 545
Cys Lys Gly Glu Ser His Trp Val Asp Asp Asp Cys Glu Glu Ile Lys
            140                 145                 150

gtt ccc gaa tgc cct gca ggg ttt gtc cgc cct ccg tta atc atc ttc 593
Val Pro Glu Cys Pro Ala Gly Phe Val Arg Pro Pro Leu Ile Ile Phe
        155                 160                 165

tct gtg gat ggt ttc cgt gca tca tac atg aag aaa ggc agc aag gtt 641
Ser Val Asp Gly Phe Arg Ala Ser Tyr Met Lys Lys Gly Ser Lys Val
    170                 175                 180

atg ccc aac att gag aaa ctg cgg tcc tgt ggc acc cat gct ccc tac 689
Met Pro Asn Ile Glu Lys Leu Arg Ser Cys Gly Thr His Ala Pro Tyr
185                 190                 195                 200

atg agg cct gtg tac ccc aca aaa acc ttc cct aat cta tat acg ctg 737
Met Arg Pro Val Tyr Pro Thr Lys Thr Phe Pro Asn Leu Tyr Thr Leu
                205                 210                 215

gcc act ggt tta tat ccg gaa tcc cat gga att gtc ggt aat tca atg 785

Ala Thr Gly Leu Tyr Pro Glu Ser His Gly Ile Val Gly Asn Ser Met
            220                 225                 230

tat gat cct gtc ttt gat gct tcg ttc cat cta cga ggg cga gag aag 833
Tyr Asp Pro Val Phe Asp Ala Ser Phe His Leu Arg Gly Arg Glu Lys
        235                 240                 245

ttt aat cat agg tgg tgg gga ggc caa ccg cta tgg att aca gcc acc 881
Phe Asn His Arg Trp Trp Gly Gly Gln Pro Leu Trp Ile Thr Ala Thr
    250                 255                 260

aag caa ggg gtg aga gct gga aca ttc ttt tgg tct gtg agc atc cct 929
Lys Gln Gly Val Arg Ala Gly Thr Phe Phe Trp Ser Val Ser Ile Pro
265                 270                 275                 280

cat gaa cgg agg atc cta acc att ctt cag tgg ctt tct ctg cca gac 977
His Glu Arg Arg Ile Leu Thr Ile Leu Gln Trp Leu Ser Leu Pro Asp
                285                 290                 295

aac gag agg cct tca gtt tat gcc ttc tac tca gag cag cct gat ttt 1025
Asn Glu Arg Pro Ser Val Tyr Ala Phe Tyr Ser Glu Gln Pro Asp Phe
            300                 305                 310

tct gga cac aag tac ggc cct ttt ggc cct gag atg aca aat cct ctg 1073
Ser Gly His Lys Tyr Gly Pro Phe Gly Pro Glu Met Thr Asn Pro Leu
        315                 320                 325

agg gag att gac aag acc gtg ggg cag tta atg gat gga ctg aaa caa 1121
Arg Glu Ile Asp Lys Thr Val Gly Gln Leu Met Asp Gly Leu Lys Gln
    330                 335                 340

ctc agg ctg cat cgc tgt gtg aac gtt atc ttt gtt gga gac cat gga 1169
Leu Arg Leu His Arg Cys Val Asn Val Ile Phe Val Gly Asp His Gly
345                 350                 355                 360

atg gaa gat gtg aca tgt gac aga act gag ttc ttg agc aac tat ctg 1217
Met Glu Asp Val Thr Cys Asp Arg Thr Glu Phe Leu Ser Asn Tyr Leu
                365                 370                 375

act aat gtg gat gac att act tta gtg cct gga act ctg gga aga att 1265
Thr Asn Val Asp Asp Ile Thr Leu Val Pro Gly Thr Leu Gly Arg Ile
            380                 385                 390

cga gcc aaa tct atc aat aat tct aaa tat gac cct aaa acc att att 1313

Arg Ala Lys Ser Ile Asn Asn Ser Lys Tyr Asp Pro Lys Thr Ile Ile
        395                 400                 405

gct aac ctc acg tgc aaa aaa ccg gat cag cac ttt aag cct tac atg 1361
Ala Asn Leu Thr Cys Lys Lys Pro Asp Gln His Phe Lys Pro Tyr Met
    410                 415                 420

aaa cag cac ctt ccc aaa cgg ttg cac tat gcc aac aac aga aga att 1409
Lys Gln His Leu Pro Lys Arg Leu His Tyr Ala Asn Asn Arg Arg Ile
425                 430                 435                 440

gaa gac atc cat tta ttg gtc gat cga aga tgg cat gtt gca agg aaa 1457
Glu Asp Ile His Leu Leu Val Asp Arg Arg Trp His Val Ala Arg Lys
                445                 450                 455

cct ttg gac gtt tat aag aaa cca tca gga aaa tgt ttt ttc cag ggt 1505
Pro Leu Asp Val Tyr Lys Lys Pro Ser Gly Lys Cys Phe Phe Gln Gly
            460                 465                 470

gac cac ggc ttt gat aac aag gtc aat agc atg cag act gtt ttc gta 1553
Asp His Gly Phe Asp Asn Lys Val Asn Ser Met Gln Thr Val Phe Val
        475                 480                 485

ggt tat ggc cca act ttt aag tac agg act aaa gtg cct cca ttt gaa 1601
Gly Tyr Gly Pro Thr Phe Lys Tyr Arg Thr Lys Val Pro Pro Phe Glu
    490                 495                 500

aac att gaa ctt tac aat gtt atg tgc gat ctc cta ggc ttg aag ccc 1649
Asn Ile Glu Leu Tyr Asn Val Met Cys Asp Leu Leu Gly Leu Lys Pro
505                 510                 515                 520

gct ccc aat aat gga act cat gga agc ttg aat cac cta ctg cgt aca 1697
Ala Pro Asn Asn Gly Thr His Gly Ser Leu Asn His Leu Leu Arg Thr
                525                 530                 535

aat acc ttt agg cca acc atg cca gac gaa gtc agc cga cct aac tac 1745
Asn Thr Phe Arg Pro Thr Met Pro Asp Glu Val Ser Arg Pro Asn Tyr
            540                 545                 550

cca ggg att atg tac ctt cag tcc gag ttt gac ctg ggc tgc acc tgt 1793
Pro Gly Ile Met Tyr Leu Gln Ser Glu Phe Asp Leu Gly Cys Thr Cys
        555                 560                 565

gac gat aag gta gag cca aag aac aaa ttg gaa gaa ctc aat aaa cgt 1841

Asp Asp Lys Val Glu Pro Lys Asn Lys Leu Glu Glu Leu Asn Lys Arg
    570                 575                 580

ctt cat acc aaa gga tca aca gaa gag aga cat ctc ctg tat gga cgg 1889
Leu His Thr Lys Gly Ser Thr Glu Glu Arg His Leu Leu Tyr Gly Arg
585                 590                 595                 600

cct gca gtg ctc tat cgg act agc tat gat atc tta tac cat acg gac 1937
Pro Ala Val Leu Tyr Arg Thr Ser Tyr Asp Ile Leu Tyr His Thr Asp
                605                 610                 615

ttt gaa agt ggt tat agt gaa ata ttc tta atg cct ctc tgg aca tcg 1985
Phe Glu Ser Gly Tyr Ser Glu Ile Phe Leu Met Pro Leu Trp Thr Ser
            620                 625                 630

tat acc att tct aag cag gct gag gtc tcc agc atc cca gaa cac ctg 2033
Tyr Thr Ile Ser Lys Gln Ala Glu Val Ser Ser Ile Pro Glu His Leu
        635                 640                 645

acc aac tgt gtt cgt cct gat gtc cgt gtg tct cca gga ttc agt cag 2081
Thr Asn Cys Val Arg Pro Asp Val Arg Val Ser Pro Gly Phe Ser Gln
    650                 655                 660

aac tgt tta gct tat aaa aat gat aaa cag atg tca tat gga ttc ctt 2129
Asn Cys Leu Ala Tyr Lys Asn Asp Lys Gln Met Ser Tyr Gly Phe Leu
665                 670                 675                 680

ttt cct ccc tac ctg agc tcc tcc cca gaa gct aag tat gat gca ttc 2177
Phe Pro Pro Tyr Leu Ser Ser Ser Pro Glu Ala Lys Tyr Asp Ala Phe
                685                 690                 695

ctc gta acc aac atg gtt cca atg tac ccc gcc ttc aaa cgt gtt tgg 2225
Leu Val Thr Asn Met Val Pro Met Tyr Pro Ala Phe Lys Arg Val Trp
            700                 705                 710

gct tat ttc caa agg gtt ttg gtg aag aaa tat gct tca gaa agg aat 2273
Ala Tyr Phe Gln Arg Val Leu Val Lys Lys Tyr Ala Ser Glu Arg Asn
        715                 720                 725

gga gtc aac gta ata agt gga ccg att ttt gac tac aat tac gat ggc 2321
Gly Val Asn Val Ile Ser Gly Pro Ile Phe Asp Tyr Asn Tyr Asp Gly
    730                 735                 740

cta cgt gac act gaa gat gaa att aaa cag tat gtg gaa ggc agc tct 2369

Leu Arg Asp Thr Glu Asp Glu Ile Lys Gln Tyr Val Glu Gly Ser Ser
745                 750                 755                 760

ata cct gtc ccc acc cac tac tac agc atc atc acc agc tgc ctg gac 2417
Ile Pro Val Pro Thr His Tyr Tyr Ser Ile Ile Thr Ser Cys Leu Asp
                765                 770                 775

ttc act cag cct gca gac aag tgt gac ggt ccc ctc tct gtg tct tcc 2465
Phe Thr Gln Pro Ala Asp Lys Cys Asp Gly Pro Leu Ser Val Ser Ser
            780                 785                 790

ttc atc ctt cct cac cga ccc gac aat gat gag agc tgt aat agc tcc 2513
Phe Ile Leu Pro His Arg Pro Asp Asn Asp Glu Ser Cys Asn Ser Ser
        795                 800                 805

gag gat gag tcg aag tgg gta gag gaa ctc atg aag atg cac aca gct 2561
Glu Asp Glu Ser Lys Trp Val Glu Glu Leu Met Lys Met His Thr Ala
    810                 815                 820

cgg gtg cgg gac att gag cac ctc act ggt ctg gat ttc tac cgg aag 2609
Arg Val Arg Asp Ile Glu His Leu Thr Gly Leu Asp Phe Tyr Arg Lys
825                 830                 835                 840

act agc cgt agc tat tcg gaa att ctg acc ctc aag aca tac ctg cat 2657
Thr Ser Arg Ser Tyr Ser Glu Ile Leu Thr Leu Lys Thr Tyr Leu His
                845                 850                 855

aca tat gag agc gag att taactttctg ggcctgggca g 2696
Thr Tyr Glu Ser Glu Ile
            860

<210> 2
<211> 862
<212> PRT
<213> Rattus norvegicus

<400> 2

Met Ala Arg Gln Gly Cys Leu Gly Ser Phe Gln Val Ile Ser Leu Phe
1               5                   10                  15

Thr Phe Ala Ile Ser Val Asn Ile Cys Leu Gly Phe Thr Ala Ser Arg

            20                  25                  30

Ile Lys Arg Ala Glu Trp Asp Glu Gly Pro Pro Thr Val Leu Ser Asp
        35                  40                  45

Ser Pro Trp Thr Asn Thr Ser Gly Ser Cys Lys Gly Arg Cys Phe Glu
    50                  55                  60

Leu Gln Glu Val Gly Pro Pro Asp Cys Arg Cys Asp Asn Leu Cys Lys
65                  70                  75                  80

Ser Tyr Ser Ser Cys Cys His Asp Phe Asp Glu Leu Cys Leu Lys Thr
                85                  90                  95

Ala Arg Gly Trp Glu Cys Thr Lys Asp Arg Cys Gly Glu Val Arg Asn
            100                 105                 110

Glu Glu Asn Ala Cys His Cys Ser Glu Asp Cys Leu Ser Arg Gly Asp
        115                 120                 125

Cys Cys Thr Asn Tyr Gln Val Val Cys Lys Gly Glu Ser His Trp Val
    130                 135                 140

Asp Asp Asp Cys Glu Glu Ile Lys Val Pro Glu Cys Pro Ala Gly Phe
145                 150                 155                 160

Val Arg Pro Pro Leu Ile Ile Phe Ser Val Asp Gly Phe Arg Ala Ser
                165                 170                 175

Tyr Met Lys Lys Gly Ser Lys Val Met Pro Asn Ile Glu Lys Leu Arg
            180                 185                 190

Ser Cys Gly Thr His Ala Pro Tyr Met Arg Pro Val Tyr Pro Thr Lys

        195                 200                 205

Thr Phe Pro Asn Leu Tyr Thr Leu Ala Thr Gly Leu Tyr Pro Glu Ser
    210                 215                 220

His Gly Ile Val Gly Asn Ser Met Tyr Asp Pro Val Phe Asp Ala Ser
225                 230                 235                 240

Phe His Leu Arg Gly Arg Glu Lys Phe Asn His Arg Trp Trp Gly Gly
                245                 250                 255

Gln Pro Leu Trp Ile Thr Ala Thr Lys Gln Gly Val Arg Ala Gly Thr
            260                 265                 270

Phe Phe Trp Ser Val Ser Ile Pro His Glu Arg Arg Ile Leu Thr Ile
        275                 280                 285

Leu Gln Trp Leu Ser Leu Pro Asp Asn Glu Arg Pro Ser Val Tyr Ala
    290                 295                 300

Phe Tyr Ser Glu Gln Pro Asp Phe Ser Gly His Lys Tyr Gly Pro Phe
305                 310                 315                 320

Gly Pro Glu Met Thr Asn Pro Leu Arg Glu Ile Asp Lys Thr Val Gly
                325                 330                 335

Gln Leu Met Asp Gly Leu Lys Gln Leu Arg Leu His Arg Cys Val Asn
            340                 345                 350

Val Ile Phe Val Gly Asp His Gly Met Glu Asp Val Thr Cys Asp Arg
        355                 360                 365

Thr Glu Phe Leu Ser Asn Tyr Leu Thr Asn Val Asp Asp Ile Thr Leu

370                 375                 380

Val Pro Gly Thr Leu Gly Arg Ile Arg Ala Lys Ser Ile Asn Asn Ser
385                 390                 395                 400

Lys Tyr Asp Pro Lys Thr Ile Ile Ala Asn Leu Thr Cys Lys Lys Pro
                405                 410                 415

Asp Gln His Phe Lys Pro Tyr Met Lys Gln His Leu Pro Lys Arg Leu
            420                 425                 430

His Tyr Ala Asn Asn Arg Arg Ile Glu Asp Ile His Leu Leu Val Asp
        435                 440                 445

Arg Arg Trp His Val Ala Arg Lys Pro Leu Asp Val Tyr Lys Lys Pro
    450                 455                 460

Ser Gly Lys Cys Phe Phe Gln Gly Asp His Gly Phe Asp Asn Lys Val
465                 470                 475                 480

Asn Ser Met Gln Thr Val Phe Val Gly Tyr Gly Pro Thr Phe Lys Tyr
                485                 490                 495

Arg Thr Lys Val Pro Pro Phe Glu Asn Ile Glu Leu Tyr Asn Val Met
            500                 505                 510

Cys Asp Leu Leu Gly Leu Lys Pro Ala Pro Asn Asn Gly Thr His Gly
        515                 520                 525

Ser Leu Asn His Leu Leu Arg Thr Asn Thr Phe Arg Pro Thr Met Pro
    530                 535                 540

Asp Glu Val Ser Arg Pro Asn Tyr Pro Gly Ile Met Tyr Leu Gln Ser

545                 550                 555                 560

Glu Phe Asp Leu Gly Cys Thr Cys Asp Asp Lys Val Glu Pro Lys Asn
                565                 570                 575

Lys Leu Glu Glu Leu Asn Lys Arg Leu His Thr Lys Gly Ser Thr Glu
            580                 585                 590

Glu Arg His Leu Leu Tyr Gly Arg Pro Ala Val Leu Tyr Arg Thr Ser
        595                 600                 605

Tyr Asp Ile Leu Tyr His Thr Asp Phe Glu Ser Gly Tyr Ser Glu Ile
    610                 615                 620

Phe Leu Met Pro Leu Trp Thr Ser Tyr Thr Ile Ser Lys Gln Ala Glu
625                 630                 635                 640

Val Ser Ser Ile Pro Glu His Leu Thr Asn Cys Val Arg Pro Asp Val
                645                 650                 655

Arg Val Ser Pro Gly Phe Ser Gln Asn Cys Leu Ala Tyr Lys Asn Asp
            660                 665                 670

Lys Gln Met Ser Tyr Gly Phe Leu Phe Pro Pro Tyr Leu Ser Ser Ser
        675                 680                 685

Pro Glu Ala Lys Tyr Asp Ala Phe Leu Val Thr Asn Met Val Pro Met
    690                 695                 700

Tyr Pro Ala Phe Lys Arg Val Trp Ala Tyr Phe Gln Arg Val Leu Val
705                 710                 715                 720

Lys Lys Tyr Ala Ser Glu Arg Asn Gly Val Asn Val Ile Ser Gly Pro

                725                 730                 735

Ile Phe Asp Tyr Asn Tyr Asp Gly Leu Arg Asp Thr Glu Asp Glu Ile
            740                 745                 750

Lys Gln Tyr Val Glu Gly Ser Ser Ile Pro Val Pro Thr His Tyr Tyr
        755                 760                 765

Ser Ile Ile Thr Ser Cys Leu Asp Phe Thr Gln Pro Ala Asp Lys Cys
    770                 775                 780

Asp Gly Pro Leu Ser Val Ser Ser Phe Ile Leu Pro His Arg Pro Asp
785                 790                 795                 800

Asn Asp Glu Ser Cys Asn Ser Ser Glu Asp Glu Ser Lys Trp Val Glu
                805                 810                 815

Glu Leu Met Lys Met His Thr Ala Arg Val Arg Asp Ile Glu His Leu
            820                 825                 830

Thr Gly Leu Asp Phe Tyr Arg Lys Thr Ser Arg Ser Tyr Ser Glu Ile
        835                 840                 845

Leu Thr Leu Lys Thr Tyr Leu His Thr Tyr Glu Ser Glu Ile
    850                 855                 860

<210> 3
<211> 20
<212> PRT
<213> Bos taurus

<400> 3

Met His Thr Ala Arg Val Arg Asp Ile Glu His Leu Thr Ser Leu Asp
1               5                   10                  15

Phe Phe Arg Lys
            20

<210>  4
<211>  29
<212>  PRT
<213>  Bos taurus

<400>  4

Tyr Ala Ser Glu Arg Asn Gly Val Asn Val Ile Ser Gly Pro Ile Phe
1               5                   10                  15

Asp Tyr Asp Tyr Asp Gly Leu His Asp Thr Glu Asp Lys
            20                  25

<210>  5
<211>  17
<212>  PRT
<213>  Bos taurus

<400>  5

Phe Asn His Arg Trp Trp Gly Gly Gln Pro Leu Trp Ile Thr Ala Thr
1               5                   10                  15

Lys